# EUROPEAN PATENT APPLICATION

(11) **EP 1 591 140 A1**
(43) Date of publication of application: **02.11.2005**
(21) Application number: 05252602.7
(22) Date of filing: 26.04.2005
(51) Int. Cl.: A61M 39/02, A61F 5/00

(54) **A surgically implantable injection port having a centered catheter connection tube**

(30) Priority: 27.04.2004 US 832773
(71) Applicant: ETHICON ENDO-SURGERY, INC., Cincinnati, Ohio 45242 (US)
(72) Inventor: Conlon, Sean P., Loveland, Ohio 45140 (US)
(74) Representative: Tunstall, Christopher Stephen

(57) **Abstract**

An implantable surgical injection port having a housing with a distal back portion having a recessed portion. The housing also includes a proximal opening and a fluid reservoir. The port also has a needle penetrable septum attached to the housing about the opening. The port further includes a catheter tube connection member in fluid communication with the reservoir. The member is attached to the recessed portion of the back portion and extends distally therefrom such that connection member does not extend distal to a distal most portion of the back portion.

## Description

### Field of the Invention

The present invention has application in conventional endoscopic and open surgical instrumentation as well as application in robotic-assisted surgery. The present invention has even further relation to adjustable surgically implantable bands, such as gastric bands for the treatment of obesity.

### Background of the Invention

The percentage of the world's population suffering from morbid obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacological methods have all been tried, and failed to correct the condition. Mechanical apparatuses for insertion into the body through non-surgical means, such as the use of gastric balloons to fill the stomach have also been employed in the treatment of the condition. Such devices cannot be employed over a long term, however, as they often cause severe irritation, necessitating their periodic removal and hence interruption of treatment. Thus, the medical community has evolved surgical approaches for treatment of morbid obesity.

Most surgical procedures for treatment of morbid obesity may generally be classified as either being directed toward the prevention of absorption of food (malabsorption), or restriction of stomach to make the patient feel full (gastric restriction) The most common malabsorption and gastric restriction technique is the gastric bypass. In variations of this technique, the stomach is horizontally divided into two isolated pouches, with the upper pouch having a small food capacity. The upper pouch is connected to the small intestine, or jejunum, through a small stoma, which restricts the processing of food by the greatly reduced useable stomach. Since food bypass much of the intestines, the amount of absorption of food is greatly reduced.

There are many disadvantages to the above procedure. Typically the above mentioned procedure is performed in an open surgical environment. Current minimally invasive techniques are difficult for surgeons to master, and have many additional drawbacks. Also, there is a high level of patient uneasiness with the idea of such a drastic procedure which is not easily reversible. In addition, all malabsorption techniques carry ongoing risks and side effects to the patient, including malnutrition and dumping syndrome.

Consequently, many patients and physicians prefer to undergo a gastric restriction procedure for the treatment of morbid obesity. One of the most common procedures involves the implantation of an adjustable gastric band. Examples of an adjustable gastric band can be found in U.S. Patents 4,592,339 issued to Kuzmak; RE 36176 issued to Kuzmak; 5,226,429 issued to Kuzmak; 6,102,922 issued to Jacobson and 5,601,604 issued to Vincent, all of which are hereby incorporated herein by reference. In accordance with current practice, a gastric band is operatively placed to encircle the stomach. This divides the stomach into two parts with a stoma in-between. An upper portion, or a pouch, which is relatively small, and a lower portion which is relatively large. The small partitioned portion of the stomach effectively becomes the patients new stomach, requiring very little food to make the patient feel full.

Once positioned around the stomach, the ends of the gastric band are fastened to one another and the band is held securely in place by folding a portion of the gastric wall over the band and closing the folded tissue with sutures placed therethrough thereby preventing the band from slipping and the encircled stoma from expanding. Gastric bands typically include a flexible substantially non-extensible portion having an expandable, inflatable portion attached thereto. The inflatable portion is in fluid communication with a remote injection site, or port. Injection or removal of an inflation fluid into or from the interior of the inflatable portion is used to adjust the size of the stoma either during or following implantation. By enlarging the stoma, the patient can eat more food without feeling as full, but will not lose weight as fast. By reducing the size of the stoma, the opposite happens. Physicians regularly adjust the size of stoma to adjust the rate of weight loss.

For most fluid injection ports used with devices such as gastric bands, the catheter tube is attached to the port via a connection tube on the side of the port. This results in the catheter tube extending laterally away from the port. This position of the catheter tube may result in the physician inadvertently puncturing the catheter tube with a needle when attempting to pierce the fluid reservoir on the port. This can cause a leak in the tube, which could necessitate an operation to fix.

One solution to this problem is described in U.S. Patent 3,310,051 issued to Schulte on December 10, 1963, which is hereby incorporated herein by reference. That reference discloses a port where the catheter connection tube, and hence the catheter, extend distally from the back side. However, that device has the catheter connection tube protruding outside of the housing of the device. This protruding catheter connection tube could pierce or other wise irritate surrounding tissue.

### Summary of the Invention

In accordance with the present invention, there is provided an implantable surgical injection port having a housing with a distal back portion having a recessed portion. The housing also includes a proximal opening and a fluid reservoir. The port also has a needle penetrable septum attached to the housing about the opening. The port further includes a catheter tube connection member in fluid communication with the reservoir. The member is attached to the recessed portion of the back portion and extends distally therefrom such that connection member does not extend distal to a distal most portion of the back portion.

### Detailed Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:

Figure 1 is a perspective view of a gastric band 101, attached to an injection port 10 made in accordance with the present invention.

Figure 3 is a perspective view of a gastric band 2 showing it in its deployed position.

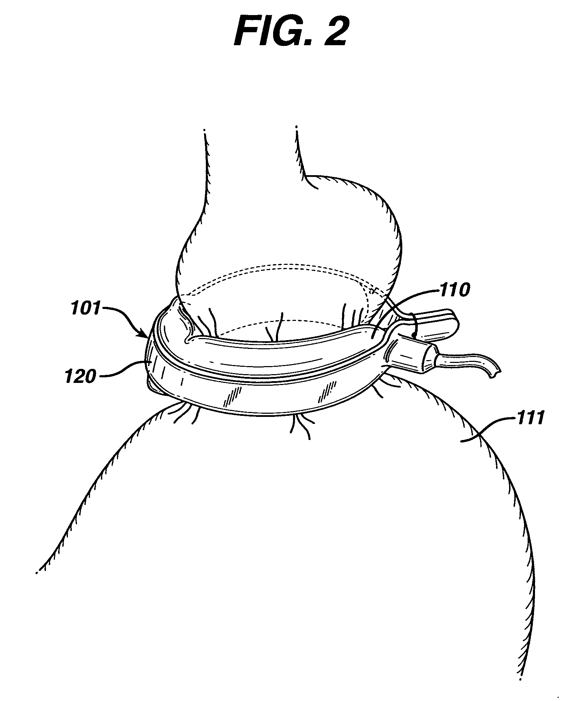

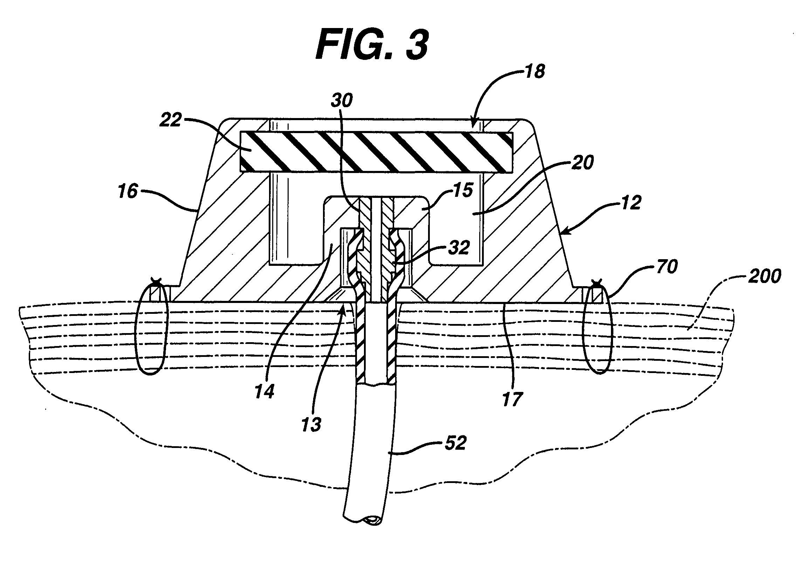

Figure 2 is a cross section of the device shown in Figure 3, taken along lines 4-4.

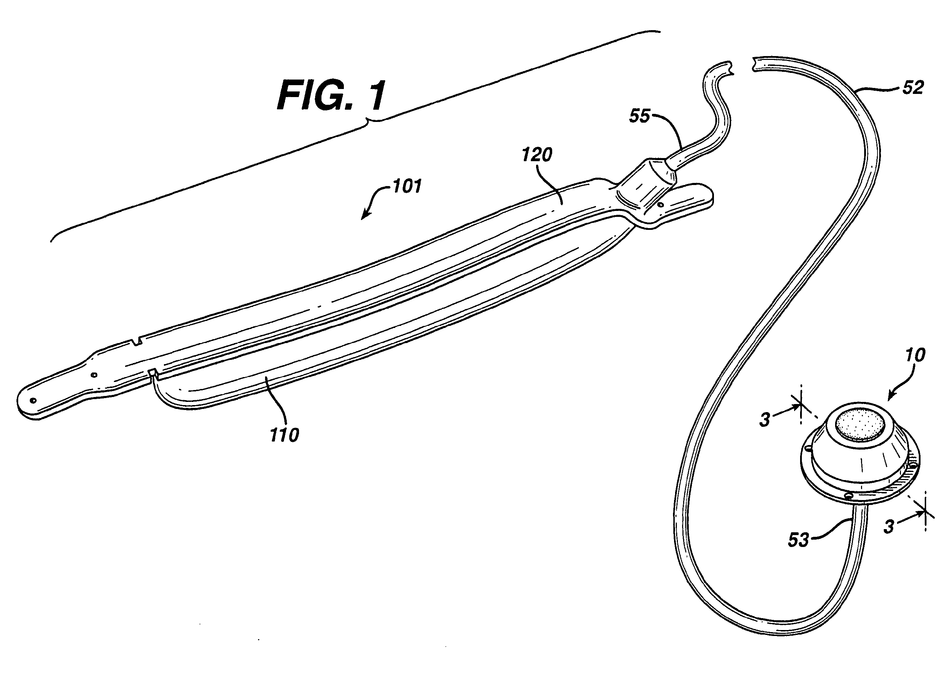

Figure 3 is a cross section view of port 10 taken along line 3-3 of Figure 1 and showing the port as being attached to the fascia of a patient.

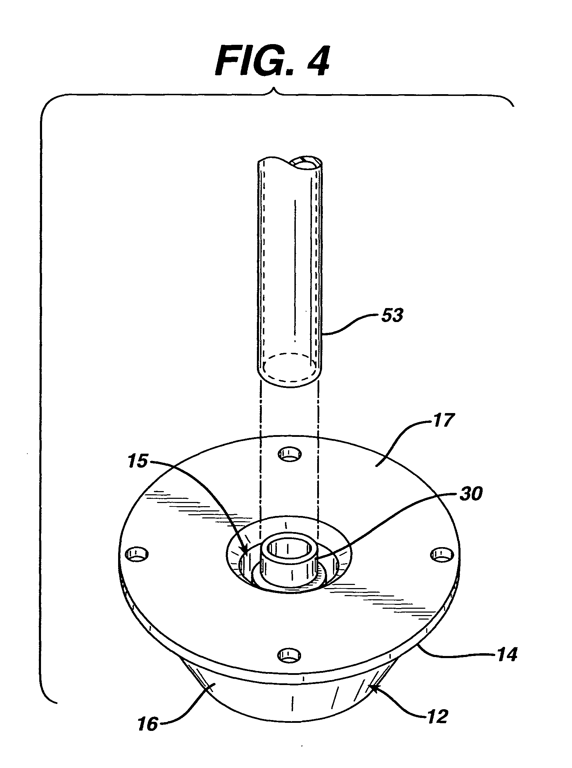

Figure 4 is a perspective view of the back portion of a port made in accordance with the present invention.

### Detailed Description of the Invention

Referring now to the drawings wherein like numerals indicate the same elements throughout the views, as stated above there is shown in Figure 1 an adjustable gastric band 101 of the type described in the above mentioned incorporated references. The band 101 includes an elongated flexible inflatable portion, alternatively referred to as balloon portion, 110 and an elongated flexible and substantially inextensible band portion 120. As seen from Figure 2, and as stated above, when the band 101 is deployed, it is positioned around the stomach 111, and the ends of the gastric band are fastened to one another.

Referring back now to Figure 1, there is shown a surgically implantable fluid injection port 10 made in accordance with the present invention. Inflatable portion 110 is in fluid communication with injection port 10 via a catheter tube 52. Tube 52 has a proximal end 53 attached to the port 10 and a distal end 55 attached to adjustable gastric band 101. Port 10 can be used for a wide range of devices in the medical field and not only for gastric bands. For example the port can also used for vascular access for drug delivery.

As seen from Figure 3, injection port 10 is implanted into a patient and attached to the fascia just below the skin of the patient, so that fluid can be inserted and withdrawn from the inflatable portion with a syringe. As seen from Figure 3 port 110 is attached to the patient via sutures 70. However, alternative means of attaching the port to the patient, such as using integral hooks, can be used as well. Such other means for attaching the port to a patient are described in commonly assigned and copending U.S. Patent Application Serial Numbers: 10/741,785 filed December 19, 2003; 60/478,763 filed December 19, 2003; 10/741,868 filed December 30, 2003; all of which are hereby incorporated herein by reference.

As seen from Figures 3 and 4, surgically implantable injection port 10 includes a housing 12. Housing 12 can be made from any number of materials including stainless steel, titanium, or polymeric materials. Housing 12 has a distal back portion 14 and a perimeter wall portion 16 extending proximally from the back portion 14 at an angle. Wall portion 16 defines a proximal opening 18, and a fluid reservoir 20 between opening 18 and back portion 14. The port includes a needle penetrable septum 22 attached to the housing about the opening 18 so as to cover the opening and seal the reservoir 20. Septum 22 can be made from any number of materials including silicone. Septum 22 is preferably placed in a proximal enough position such that the depth of the reservoir 22 is sufficient enough to expose the open tip of a needle, such as a Huber needle, so that fluid transfer can take place. Septum 22 is preferably arranged so that it will self seal after being punctured by a needle and the needle is withdrawn. In one embodiment, the septum is made from silicone which is under compression when attached to the housing.

Port 10 further includes a catheter tube connection member 30, in fluid communication with reservoir 20, which is attached to the back portion 14, preferably it its center 13, of the housing 12 and extends distally from the reservoir 20. This distally extending arrangement eliminates the problem of a catheter tube extending radially from the reservoir, where it could be punctured by a physician. Member 30 can be a separate piece which is welded interference fitted, screw threaded, glued or otherwise attached to back portion 14, or it could be integral, i.e. molded, with back portion 14. For the embodiment shown in Figure 3, back portion 14 includes a recessed portion in which the connection member 30 is attached to. Recessed portion 15 allows the connection member to extend distally from reservoir 20 such that it does not extend distal to a distal most portion 17 of back portion 14. The advantages of having the connection member 30 completely within the housing are that the member will not erode any tissue, and the tube will stay perpendicular to the bottom surface of the port and not be subject to any bending loads which could eventually cause failure of the tube.

Connection member 30, As shown in Figure 3, includes one or more radially extending flanges 32 extending therefrom. Flange 32 preferably has a diameter greater than the relaxed diameter of the catheter tube 52 (typically made from silicone or other polymeric materials). This is so that tube 52 elastically expands to fit over flange 32 so as to provide a better interference fit and give a good fluid tight seal between tube 52 and member 30.

In practice, a physician would attach the port 10 to the patient. Thereafter, he/she would attach a catheter tube at the back portion of the device so that the catheter tube is in fluid communication with the reservoir and extends distally from the reservoir. This eliminates the problem of a catheter tube extending radially from the reservoir, where it could be punctured by a physician or kinked from bending through a 90 degree bend into the patient.

It will become readily apparent to those skilled in the art that the above invention has equally applicability to other types of implantable bands. For example, bands are used for the treatment of fecal incontinence. One such band is described in U.S. Patent 6,461,292 which is hereby incorporated herein by reference. Bands can also be used to treat urinary incontinence. One such band is described in U.S. Patent Application 2003/0105385 which is hereby incorporated herein by reference. Bands can also be used to treat heartburn and/or acid reflux. One such band is described in U.S. Patent 6,470,892 which is hereby incorporated herein by reference. Bands can also be used to treat impotence. One such band is described in U.S. Patent Application 2003/0114729 which is hereby incorporated herein by reference.

While preferred embodiments of the present invention have been shown and described herein, it will be obvious to those skilled in the art that such embodiments are provided by way of example only. Numerous variations, changes, and substitutions will now occur to those skilled in the art without departing from the invention. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the spirit and scope of the appended claims.

## Claims

1. An implantable surgical injection port comprising:
a. a housing having a distal back portion having a recessed portion, a proximal opening and a fluid reservoir therebetween;
b. a needle penetrable septum attached to said housing about said opening; and
c. a catheter tube connection member in fluid communication with said reservoir, said member is attached to said recessed portion of said back portion and extends distally therefrom such that connection member does not extend distal to a distal most portion of said back portion.

2. The injection port of claim 1 wherein said catheter tube connection member is attached to a center of said back portion.

3. The injection port of claim 1 wherein said catheter tube connection member includes a radially extending flange.

4. The injection port of claim 3 further including a catheter tube wherein said catheter tube has an inner diameter less than an outer diameter of said radially extending flange.

5. The injection port of claim 1 further including a catheter tube wherein said catheter tube is attached to said member and extends distally therefrom.

6. The injection port of claim 1 wherein said housing comprises titanium.

7. The injection port of claim 1 wherein said septum self seals after being punctured by a needle and the needle is withdrawn.

8. The injection port of claim 1 wherein said septum comprises silicone.

9. The injection port of claim 1 wherein said injection port further includes a means for attaching said port to a patient.

10. An implantable surgical injection port comprising:
a. a housing having a distal back portion having a recessed portion, a proximal opening and a fluid reservoir therebetween;
b. a needle penetrable septum attached to said housing about said opening;
c. a catheter tube connection member in fluid communication with said reservoir, said member is attached to said recessed portion of said back portion and extends distally therefrom such that connection member does not extend distal to a distal most portion of said back portion; and
d. a catheter tube attached to said connection member and extending distally therefrom.

11. An implantable surgical injection port comprising:
a. a housing having a distal back portion, a proximal opening and a fluid reservoir therebetween;
b. a needle penetrable septum attached to said housing about said opening;
c. a catheter tube connection member in fluid communication with said reservoir, said member is attached to said back portion and extends distally from said reservoir; and
d. a catheter tube having a proximal end attached to said connection member and a distal end extending therefrom and attached to an adjustable gastric band.
